# EUROPÄISCHE PATENTSCHRIFT

(11) **EP 2 821 055 B1**
(45) Veröffentlichungstag und Bekanntmachung des Hinweises auf die Patenterteilung: **03.05.2017**
(21) Anmeldenummer: 14158140.5
(22) Anmeldetag: 06.03.2014
(51) Int. Cl.: A61K 8/44, A61K 8/46, A61Q 11/00, A61K 8/73

(54) **Mund- und Zahnpflege- und Reinigungsmittel mit optimiertem Schaumverhalten und verbesserter Wirkstoffdeposition**
Oral and tooth care and cleaning agent with optimised foaming characteristics and improved active ingredient deposition
Produit de nettoyage de la bouche et des dents avec effet moussant optimisé et dépôt d'agent actif amélioré

(30) Priorität: 02.07.2013 DE 102013212874
(43) Veröffentlichungstag der Anmeldung: 07.01.2015
(73) Patentinhaber: Henkel AG & Co. KGaA, 40589 Düsseldorf (DE)
(72) Erfinder: WEIß, Thomas, 40591 Düsseldorf (DE); DUSCHEK, Nicole, 40595 Düsseldorf (DE); MIEHLICH, Kristin, 42119 Wuppertal (DE); KIRMIS, Jürgen, 41472 Neuss (DE)

(56) Entgegenhaltungen:
- DE-A1-102011 082 433
- US-A1- 2006 134 018
- US-A1- 2009 271 936
- US-A1- 2012 244 203
- DATABASE GNPD [Online] MINTEL; 31. Dezember 1999 (1999-12-31), "AZ synergy", XP002732681, Database accession no. 15827
- Anonymous: "AQUALON®SodiumCarboxymethylcellulose", Brenntag Specialties , 1999, XP002732682, Gefunden im Internet: URL:http://www.brenntagspecialties.com/en/ downloads/Products/Multi_Market_Principals /Aqualon/Aqualon_CMC_Booklet.pdf [gefunden am 2014-11-19]

## Beschreibung

Die Anmeldung betrifft Mund- und Zahnpflege sowie Mund- und Zahnreinigungsmittel enthaltend eine spezielle Kombination aus Polymeren, anionischen und amphoteren Tensiden. Weiterhin betrifft die Anmeldung ein kosmetisches Verfahren zur Reinigung der Zähne bei gleichzeitig optimierter Wirkstoffdeposition.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.

Neben der Reinigung der Zähne erwartet der Verbraucher von den gattungsgemäßen Produkten auch eine Pflege der Zähne und der Mundhöhle. So sind insbesondere ein "sauberes" Gefühl, d.h. eine glatte und glänzende Zahnoberfläche sowie ein frisches Gefühl im Mund wesentliche Aspekte für den Kaufanreiz von Zubereitungen zur Mund- und Zahnpflege- und -reinigung. Ein erfolgreiches Mittel der gattungsgemäßen Art sollte daher die Zähne gründlich reinigen, ohne den Zahn oder die Zahnoberfläche zu schädigen.

Zahnpasten entfalten ihre Reinigungsleistung hauptsächlich durch die in der Zahnpasta enthaltenen Poliermittel, in geringerem Maße auch durch die enthaltenen Tenside. Die Poliermittel müssen, um ihre Reinigungs- und Polierwirkung zu entfalten, eine gewisse Abrasivität gegenüber der Zahnoberfläche aufweisen. Es ist jedoch von größter Bedeutung, daß die Abrasivität gegenüber Zahnschmelz und Dentin auf niedrigen Werten gehalten wird, um eine Schädigung der Zahnoberfläche durch den täglichen Gebrauch der Zahnpasta zu vermeiden. Vor allem dürfen die verwendeten Abrasiva keinen unnötigen Abtrag des Zahnschmelzes (Enamel) bewirken, und auch das darunterliegende, weichere Dentin möglichst nicht schädigen, da bei Personen mit schmerzempfindlichen Zähnen häufig die Ursache in einer dünnen Enamelschicht oder freiliegenden Zahnhälsen liegt.

Der Mundraum ist mit einer befeuchteten Schleimhaut (Mucosa) ausgekleidet. Diese besteht generell aus zwei Lagen: einem mehrschichtigen, in der Regel unverhornten Plattenepithel und dem darunter liegenden Bindegewebe. Im Gegensatz zur normalen Haut besitzt die Schleimhaut keine Hornschicht. Als Zahnfleisch wird der epitheliale Bestandteil des Zahnhalteapparates bezeichnet. Auch dieses besteht aus einem mehrschichtigen Plattenepithel, welches ebenfalls nur wenige Hornschichten aufweist.

Ein wichtiges Charakteristikum von Mundschleimhaut und Zahnfleisch ist demzufolge das Fehlen bzw. die sehr schwache Ausbildung der Hornschicht. Mit dieser Hornschicht fehlt der gesamten Mundregion auch eine wichtige äußere Barriere gegenüber mechanischen, chemischen und physikalischen Einflüssen. Die Schleimhautzellen sind somit allen vorgenannten Beanspruchungen ohne Schutzschicht ausgesetzt und reagieren aus diesem Grund empfindlicher auf die durch das Zähneputzen hervorgerufenen mechanischen Beanspruchungen und die mit der Anwendung von Zahncremes verbundenen chemischen Reize.

Der vorliegenden Anmeldung lag daher die Aufgabe zugrunde, ein sowohl für die Zähne als auch für das Zahnfleisch bzw. die Mundschleimhaut optimiertes Zahnreinigungs- und Pflegemittel bereitzustellen, welches den Einsatz der für Zahnreinigung und Kariesprophylaxe unverzichtbaren Inhaltsstoffe wie Abrasiva und Tenside ermöglicht, hierbei aber gleichzeitig deren negativen Einfluss auf das Zahnfleisch minimiert und geeignet ist, die Deposition von Wirkstoffen zu verbessern, um weitere Effekte zu erzielen.

Zahnschonenende Zahnpasten mit guter Putzaktivität sind im Stand der Technik bereits beschrieben worden. So offenbart die US 2012/244203 A1 Zahnpasten, welche 1 Gew.-% Natriumlaurylsulfat, 0,3 bis 0,5 Gew.-% Cocoamidopropylbetain, 0,7 Gew.-% CMC und 0,7 Gew.-% Xanthan enthalten. Auch die US 2006/134018 A1 offenbart Zahnpasten, enthaltend Natriumlaurylsulfat, Cocoamidopropylbetain,CMC und Xanthan.

Die US 2009/271936 A1 offenbart mittels elektrischer Zahnbürsten zu applizierende Zahnpasten, die 3,5 Gew.-% Natriumlaurylsulfat, 2,5 Gew.-% Cocoamidopropylbetain, 0,1 Gew.-% CMC und 0,4 Gew.-% Xanthan enthalten.

Parallel zu den Entwicklungen auf Produktseite sind auch auf gerätetechnischer Seite Weiterentwicklungen verfolgt worden. Neben der herkömmlichen Handzahnbürste, die vom Verbraucher mit kreisenden Bewegungen im Mund bewegt wird, haben sich elektrische Zahnbürsten im Markt etabliert, die einen Teil der Bewegung der Borsten auf der Zahnoberfläche durch eine im Handstück befindliche - meist wiederaufladbare - Batterie erzeugen. Der Verbraucher kann hierdurch je nach Modell die kreisende Handbewegung durch eine horizontal-lineare Bewegung ersetzen.

Elektrisch bewegte Bürstenköpfe sind dabei in vielfältigen Ausgestaltungen marktüblich. So existieren runde Bürstenköpfe, die elektrisch in Rotation oder Teilrotation mit Drehungen im und gegen den Uhrzeigersinn versetzt werden. Es gibt aber auch länglich, der herkömmlichen Zahnbürste nachempfundene Bürstenköpfe, die elektrisch oszillieren.

Allen elektrischen Zahnbürsten ist der Vorteil gemein, daß sie den Verbraucher anteilsweise von bestimmten Bewegungsabläufen entlasten und im Ruf stehen, die Zähne gründlicher zu reinigen. Diesen Vorteilen stehen aber auch Nachteile gegenüber: Eine elektrische Zahnbürste führt auf der Zahnoberfläche üblicherweise zu einer intensiveren Belastung als beim Zähneputzen per Hand. Werden Art und Gehalt der Schleifkörper in der Zahncreme nicht hierauf abgestimmt, können in Verbindung mit einer elektrischen Zahnbürste Schaden am Zahnschmelz entstehen.

Ein weiteres Problem besteht darin, daß die aufwendige Mechanik im Bürstenkopf verklebt werden kann und die Zahnbürste dann die entsprechende elektrische Bewegung nicht mehr durchführen kann. Zusätzlich muß auch die Rheologie beachtet werden, denn auf den sich bewegenden Bürstenköpfen muß eine bestimmte Haftfähigkeit gegeben sein, um Verspritzen der Zahncreme zu vermeiden. Auch muß die Schaumart und -menge auf den Einsatz mit elektrischen Zahnbürsten abgestimmt werden. Im Gegensatz zur Anwendung mit Handzahnbürsten benötigen Zahncremes für elektrische Zahnbürsten einen stabileren und feinporigeren Schaum, damit die höheren mechanischen Belastungen nicht zu schnell zu einem Brechen des Schaumes führen.

Es ist daher weiterhin die Aufgabe dieser Erfindung, Zahnreinigungsmittel bereitzustellen, welche auch mit elektrischen Zahnbürsten erfolgreich applizierbar sind, ohne dabei zu kleckern oder zu tropfen oder die Mechanik der Zahnbürste zu beeinträchtigen.

In nicht vorhersehbarer Weise wurde nun gefunden, daß die zuvor beschriebenen Aufgaben durch eine Wirkstoffkombination aus Tensiden zwei Tensidklassen und zwei Polymeren gelöst werden kann. Die Polymere bewirken in Kombination mit den Tensiden einen stabilen und feinporigen Schaum, der die Deposition von Aktivstoffen auf den Zahnschmelz sowie auf Zahnfleisch und Mundschleimhaut intensiviert. Dabei ist der Schaum stabil und leicht auswaschbar, so daß die Applizierbarkeit mittels elektrischer Zahnbürsten in besonderem Maße gegeben ist.

Gegenstand der vorliegenden Erfindung ist in einer ersten Ausführungsform ein Mund- und Zahnpflege- und -reinigungsmittel, enthaltend - bezogen auf sein Gewicht -
a) 0,05 bis 5,0 Gew.-% Natriumlaurylsulfat;
b) 0,05 bis 5,0 Gew.-% Cocamidopropylbetain;
c) 0,1 bis 10 Gew.-% Carboxymethylcellulose;
d) 0,1 bis 10 Gew.-% Xanthan,
wobei das Gewichtsverhältnis von anionischen Tensiden zu amphoteren Tensiden bei ≥ 4:1 liegt.

Entsprechend konzipierte Mittel reinigen die Zähne in bewährter Weise und gewährleisten eine optimale Kariesprophylaxe, üben hierbei jedoch keine nachteiligen Einflüsse auf Zahnfleisch und Mundschleimhaut aus, da sie das Zahnfleisch bzw. die Mundschleimhaut vitalisieren und deren Widerstandsfähigkeit stärken. Zudem sind Zahnfleisch und Mundschleimhaut gegenüber mechanischen Beanspruchungen, wie sie durch den Zahnreinigungsvorgang hervorgerufen werden, besser geschützt und schneller regenerationsfähig.

Unter Vitalisierung wird im Sinne der vorliegenden Erfindung eine Erhöhung der Vitalität der Zellen des Zahnfleischs und der Mundschleimhaut verstanden. Eine Erhöhung der Vitalität bedeutet eine Erhöhung der Zellüberlebensfähigkeit, welche beispielsweise nach verschiedenen Verfahren wie dem Nachweis der Zellatmungsaktivität mittels Vitalstoffen ermittelt werden kann. Die Erhöhung der Vitalität einer Zelle verbessert auch deren Widerstandsfähigkeit.

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes, Mund- und Zahnspülungen sowie Mund- und Zahngele. Bevorzugt geeignet sind Zahnpasten und flüssige Zahnreinigungsmittel. Hierzu können die Mund- und Zahnpflege- und reinigungsmittel z.B. in Form von Zahnpasten, flüssigen Zahncremes, Zahnpulvern, Mundwässern oder gegebenenfalls auch als Kaumasse, z. B. als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden. Eine weitere besonders bevorzugte Ausführungsform der vorliegenden Erfindung sind Mundspüllösungen und Mundwasser, die zum Ausspülen der Mundhöhle verwendet werden.

Als ersten wesentlichen Bestandteil enthalten die erfindungsgemäßen Mund und Zahnpflege- und - reinigungsmittel 0,05 bis 5,0 Gew.-% Natriumlaurylsulfat.

Erfindungsgemäß bevorzugte Mund und Zahnpflege- und -reinigungsmittel sind dadurch gekennzeichnet, daß sie 0,1 bis 4,0 Gew.-%, vorzugsweise 0,75 bis 3,0 Gew.-%, besonders bevorzugt 1,0 bis 2,5 Gew.-%, weiter bevorzugt 1,25 bis 2,0 Gew.-% und insbesondere 1,5 bis 1,8 Gew.-% Natriumlaurylsulfat enthalten.

Als zweiten wesentlichen Bestandteil enthalten die erfindungsgemäßen Mund und Zahnpflege- und - reinigungsmittel 0,05 bis 5,0 Gew.-% Cocamidopropylbetain.

In einer besonders bevorzugten Ausführungsform ist ein erfindungsgemäßes Mund- und Zahnpflege- und -reinigungsmittel daher dadurch gekennzeichnet, dass es, bezogen auf sein Gesamtgewicht, 0,1 bis 4,0 Gew.-%, vorzugsweise 0,2 bis 3,0 Gew.-%, besonders bevorzugt 0,3 bis 2,5 Gew.-%, weiter bevorzugt 0,4 bis 2,0 Gew.% und insbesondere 0,5 bis 1,0 Gew.-% Cocamidopropylbetain enthält.

Es hat sich für die Schaumstabilität und Wirkstoffdeposition als bevorzugt herausgestellt, wenn anionische Tenside mindestens in gleicher Menge enthalten sind wie amphotere Tenside. Besonders bevorzugt werden anioische Tenside im Überschuß eingesetzt, wobei bevorzugte erfindungsgemäße Mund und Zahnpflege- und -reinigungsmittel dadurch gekennzeichnet sind, daß das Gewichtsverhältnis von anionischen Tensiden zu amphoteren Tensiden bei ≥ 1:1, vorzugsweise bei ≥ 2,5:1, besonders bevorzugt bei ≥ 4:1, weiter bevorzugt bei ≥ 5:1, und insbesondere bei ≥ 7:1 liegt. Als dritten wesentlichen Bestandteil enthalten die erfindungsgemäßen Mund und Zahnpflege- und - reinigungsmittel 0,1 bis 10 Gew.-% Carboxymethylcellulose;

Die in erfindungsgemäßen Mund- und Zahnpflege- und -reinigungsmitteln eingesetzte Carboxymethylcellulose wird durch chemische Umsetzung (Veretherung) von Cellulose hergestellt. Durch die Steuerung der Reaktionsbedingungen ist es möglich, den Grad der Veretherung genau einzustellen. Als Maß für den Veretherungsgrad wird der Substitutionsgrad (Abkürzung DS) angegeben. Der Substitutionsgrad gibt die durchschnittliche Anzahl der veretherten Hydroxylgruppen an einer Glucoseeinheit an. Da eine Glucoseeinheit drei Hydroxylgruppen für eine Umsetzung zur Verfügung hat, beträgt der maximal erreichbare Substitutionsgrad DS = 3.

Es hat sich gezeigt, daß die Fluoriddeposition weiter verbessert wird, wenn die eingesetzte Carboxymethylcellulose Substitutionsgrade von 0,5 bis 2,5, vorzugsweise von 0,65 bis1,45, aufweist. Entsprechende erfindungsgemäße Produkte, die CMCs der genannten Substitutionsgrade enthalten, sind erfindungsgemäß besonders bevorzugt.

Eine weitere Möglichkeit der Verbesserung der Fluoriddeposition besteht darin, den erfindungsgemäßen Mund und Zahnpflege- und -reinigungsmittel ein definiertes rheologisches Profil Diese Tenside werden nach der INCI-Nomenklatur als Amidopropylbetaine bezeichnet, wobei die Vertreter, die sich von Kokosfettsäuren ableiten, bevorzugt sind und als Cocoamidopropylbetaine bezeichnet werden. Besonders bevorzugt werden erfindungsgemäß Tenside der Formel (Bet-I) eingesetzt, die ein Gemisch der folgenden Vertreter sind:
H₃C-(CH₂)₇-C(O)-NH-(CH₂)₃N⁺(CH₃)₂CH₂COO⁻
H₃C-(CH₂)₉-C(O)-NH-(CH₂)₃N⁺(CH₃)₂CH₂COO⁻
H₃C-(CH₂)₁₁-C(O)-NH-(CH₂)₃N⁺(CH₃)₂CH₂COO⁻
H₃C-(CH₂)₁₃-C(O)-NH-(CH₂)₃N⁺(CH₃)₂CH₂COO⁻
H₃C-(CH₂)₁₅-C(O)-NH-(CH₂)₃N⁺(CH₃)₂CH₂COO⁻
H₃C-(CH₂)₇-CH=CH-(CH₂)₇-C(O)-NH-(CH₂)₃N⁺(CH₃)₂CH₂COO⁻

In einer weiteren besonders bevorzugten Ausführungsform ist ein erfindungsgemäßes Mund- und Zahnpflege- und -reinigungsmittel daher dadurch gekennzeichnet, dass es, bezogen auf sein Gesamtgewicht, 0,1 bis 4,0 Gew.%, vorzugsweise 0,2 bis 3,0 Gew.-%, besonders bevorzugt 0,3 bis 2,5 Gew.-%, weiter bevorzugt 0,4 bis 2,0 Gew.-% und insbesondere 0,5 bis 1,0 Gew.-% Cocamidopropylbetain enthält.

Es hat sich für die Schaumstabilität und Wirkstoffdeposition als bevorzugt herausgestellt, wenn anionische Tenside mindestens in gleicher Menge enthalten sind wie amphotere Tenside. Besonders bevorzugt werden anioische Tenside im Überschuß eingesetzt, wobei bevorzugte erfindungsgemäße Mund und Zahnpflege- und -reinigungsmittel dadurch gekennzeichnet sind, daß das Gewichtsverhältnis von anionischen Tensiden zu amphoteren Tensiden bei ≥ 1:1, vorzugsweise bei ≥ 2,5:1, besonders bevorzugt bei ≥ 4:1 , weiter bevorzugt bei ≥ 5:1 , und insbesondere bei ≥ 7:1 liegt. Als dritten wesentlichen Bestandteil enthalten die erfindungsgemäßen Mund und Zahnpflege- und - reinigungsmittel 0,1 bis 10 Gew.-% Carboxymethylcellulose;

Die in erfindungsgemäßen Mund- und Zahnpflege- und -reinigungsmitteln eingesetzte Carboxymethylcellulose wird durch chemische Umsetzung (Veretherung) von Cellulose hergestellt. Durch die Steuerung der Reaktionsbedingungen ist es möglich, den Grad der Veretherung genau einzustellen. Als Maß für den Veretherungsgrad wird der Substitutionsgrad (Abkürzung DS) angegeben. Der Substitutionsgrad gibt die durchschnittliche Anzahl der veretherten Hydroxylgruppen an einer Glucoseeinheit an. Da eine Glucoseeinheit drei Hydroxylgruppen für eine Umsetzung zur Verfügung hat, beträgt der maximal erreichbare Substitutionsgrad DS = 3.

Es hat sich gezeigt, daß die Fluoriddeposition weiter verbessert wird, wenn die eingesetzte Carboxymethylcellulose Substitutionsgrade von 0,5 bis 2,5, vorzugsweise von 0,65 bis1,45, aufweist. Entsprechende erfindungsgemäße Produkte, die CMCs der genannten Substitutionsgrade enthalten, sind erfindungsgemäß besonders bevorzugt.

Eine weitere Möglichkeit der Verbesserung der Fluoriddeposition besteht darin, den erfindungsgemäßen Mund und Zahnpflege- und -reinigungsmittel ein definiertes rheologisches Profil zu geben. In erster Linie kann dies durch den Einsatz von CMCs erfolgen, die ihrerseits definierte rheologische Profile haben.

Viskositäten von CMC-Lösungen werden beispielsweise mit Hilfe von Brookfield Viskosimetern, Rotationsviskosimetern oder Höppler Viskosimetern bestimmt. Die Viskositäten werden in Pas oder mPa·s angegeben. Je nach Methode können sich die angegebenen Viskositäten deutlich unterscheiden. Erfindungsgemäß bevorzugt ist der Einsatz von CMCs, welche in einer 2 Gew.-%igen wäßrigen Lösung bei 20°C folgende Viskositäten aufweisen:
- 1.000 bis 100.000 mPas, vorzugsweise 2.000 bis 50.000 mPas und insbesondere 5.000 bis 25.000 mPa·s mit einem Rotationsviskosimeter bei einer Scherrate von 2,55 1/s
- 5.000 bis 100.000 mPas, vorzugsweise 10.000 bis 70.000 mPas und insbesondere 35.000 bis 50.000 mPa·s mit einem Brookfield LVT Viskosimeter
- 50.000 bis 250.000 mPas, vorzugsweise 75.000 bis 200.000 mPas und insbesondere 120.000 bis 140.000 mPas mit einem Höppler Viskosimeter

Die Viskosität einer wässrigen CMC-Lösung ist demnach keine absolute Größe, sondern wird stark von den Messparametern bestimmt.

Bezogen auf das anwendungsfertige erfindungsgemäße Mittel sind solche Mund- und Zahnpflege- und -reinigungsmittel bevorzugt, die bei 20°C eine Viskosität (gemessen mit Brookfield Synchro-Lectric Viskosimeter, Typ RVT mit Helipath-Stativ, Spindel 3 und 20 U/min) von 100 bis 1000 Pas (100.000 bis 1.000.000 mPas), vorzugsweise von 200 bis 750 Pas (200.000 bis 750.000 mPas), weiter bevorzugt von 350 bis 650 Pas (350.000 bis 650.000 mPas) und insbesondere von 450 bis 550 Pas (450.000 bis 550.000 mPas) aufweisen.

Die Menge an CMC, die in bevorzugten erfindungsgemäßen Mitteln eingesetzt wird, liegt typischerweise oberhalb 0,1 Gew.-% und unterhalb 10 Gew.-%. Bevorzugte erfindungsgemäße Mund- und Zahnpflege- und -reinigungsmittel sind dadurch gekennzeichnet, daß sie - bezogen auf ihr Gewicht - 0,2 bis 7,5 Gew.-%, vorzugsweise 0,25 bis 5 Gew.-%, besonders bevorzugt 0,3 bis 4 Gew.%, weiter bevorzugt 0,4 bis 3 Gew.-%, noch weiter bevorzugt 0,45 bis 2 Gew.-% und insbesondere 0,5 bis 0,8 Gew.-% Carboxymethylcellulose enthalten.

Als vierten wesentlichen Bestandteil enthalten die erfindungsgemäßen Mund und Zahnpflege- und - reinigungsmittel 0,1 bis 10 Gew.-% Xanthan.

Xanthan bzw. Xanthangummi ist ein natürlicher, nachwachsender Rohstoff und wird als ein anionisches Polysaccharid vom Bakterium *Xanthomonas campestris* ausgeschieden.

Das Molekülgewicht des eingesetzten Xanthangummis beträgt bevorzugt 2·10⁶ bis 20·10⁶ g/mol.

Als Molekülbausteine enthält Xanthangummi D-Glucose, D-Mannose, D-Glucuronsäure, Acetat und Pyruvat in einem ungefähren molaren Verhältnis von 28 zu 30 zu 20 zu 17 zu 5,1 bis 6,3. Das

Polymerrückgrat des Xanthangummis bildet sich aus einer Cellulose-Kette aus β-1,4-gebundenen Glucoseeinheiten.

Xanthan enthält Struktureinheiten der folgenden Formel

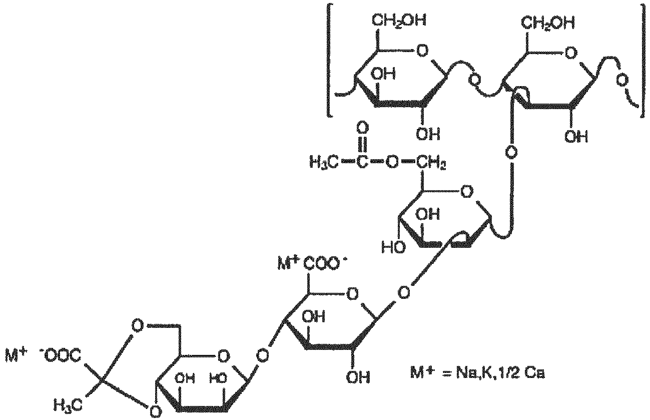

Bevorzugte erfindungsgemäße Mund- und Zahnpflege- und -reinigungsmittel enthalten - bezogen auf ihr Gewicht - 0,15 bis 5 Gew.-%, vorzugsweise 0,2 bis 2,5 Gew.-%, besonders bevorzugt 0,25 bis 1 Gew.-%, weiter bevorzugt 0,3 bis 0,75 Gew.-%, noch weiter bevorzugt 0,35 bis 0,6 Gew.-% und insbesondere 0,4 bis 0,5 Gew.-% Xanthan.

Erfindungsgemäße bevorzugt geeignete Xanthane weist in einer 1 Gew.-%-igen wässrigen KCI-Lösung eine Viskosität von mindestens 1200 bis 1600 mPa.s (Brookfield DV-I Viskosimeter, Spindel #6 bei 23°C und 10 U/min) auf.

Eine besonders effektive Kombination der beiden Polymere ist diejenige, in denen das eine Polymer maximal in doppelt so hoher Menge enthalten ist wie das andere Polymer. Bevorzugt wird mindestens genauso viel CMC eingesetzt wird wie Xanthan. Besonders bevorzugt wird CMC im leichten Überschuß zu Xanthan eingesetzt, da dann besonders gute Schaumstabilitäten erzielt werden und die Mittel besonders effektiv Zahnschmelz und Zahnfleisch schützen.

Erfindungsgemäß besonders bevorzugte Mund- und Zahnpflege- und -reinigungsmittel sind dadurch gekennzeichnet, daß das Gewichtsverhältnis von Carboxymethylcellulose zu Xanthan zwischen 2:1 und 1:2, vorzugsweise zwischen 1,75:1 und 1:1,5, besonders bevorzugt zwischen 1,5:1 und 1:1,25 und insbesondere zwischen 1,2:1 und 1:1 liegt.

Die erfindungsgemäße Kombination aus anionischem und amphoterem Tensid und den beiden Polymeren ist schonend für das Zahnfleisch und stellt gleichzeitig eine ausreichende Reinigung der Zahnoberflächen sicher.

Für die Wirkung der erfindungsgemäßen Mittel hat es sich als vorteilhaft erwiesen, die zuvor beschriebene Wirkstoffkombination aus anionischem Tensid und amphoterem Tensid durch weitere spezifische Wirkstoffe zu ergänzen, wobei sich wundheilende und entzündungshemmende Stoffe und Fluoridverbindungen als besonderes wirkungsvoll erwiesen haben.

Die erfindungsgemäße Kombination kann ihre vorteilhaften Wirkungen auf das Zahnfleisch und die Mundschleimhaut durch den Einsatz von Vitamin E noch weiter steigern.

Als weiteren Inhaltsstoff enthalten bevorzugte erfindungsgemäße Mund- und Zahnpflege- und -reinigungsmittel daher gegebenenfalls acetyliertes Vitamin E. Im Zuge der zu dieser Erfindung führenden Arbeiten hat sich herausgestellt, dass sich das bzw. die erfindungsgemäßen Mittel insbesondere dann besonders vitalisierend auf Zahnfleisch und Mundschleimhaut auswirken, wenn das gegebenenfalls acetylierte Vitamin E in bestimmten Konzentrationsbereichen eingesetzt wird. Mund- und Zahnpflege- und -reinigungsmittel, die, bezogen auf ihr Gesamtgewicht, 0,01 bis 1,0 Gew.-%, vorzugsweise 0,05 bis 0,5 Gew.-% gegebenenfalls acetyliertes Vitamin E enthalten, sind aus diesem Grund erfindungsgemäß bevorzugt.

Die Bezeichnung "gegebenenfalls acetyliertes Vitamin E" umfasst eine Reihe wirksamer Substanzen. Zu diesen Substanzen zählen insbesondere die Substanzfamilien der Tocopherole, der Tocotrienole sowie die Tocomonoenole und marinen Tocopherole (MDT, marine derived tocopherols).

Allen Vitamin-E-Formen gemeinsam ist ein an Position 6 hydroxyliertes Chromanring-Grundgerüst. Die vier oben genannten Substanzfamilien unterscheiden sich hinsichtlich unterschiedlich gesättigter Seitenketten, wobei
- die Tocopherole eine gesättigte Seitenkette aufweisen;
- die Tocomonoenole und marinen Tocopherole eine einfach ungesättigte Seitenketten aufweisen; und
- die Tocotrienole eine dreifach gesättigte Seitenkette aufweisen.

In Abhängigkeit von der Methylierung des Chroman-Grundgerüsts wird in jeder der Substanzfamilien zwischen α-, β-, χ- und δ-Formen unterschieden.

So umfasst die Substanzfamilie der Tocopherole α-Tocopherol, β-Tocopherol, χ-Tocopherol, δ-Tocopherol sowie die weiteren natürlich vorkommenden Tocopherole 5,7-Dimethyltocol und 7-Methyltocol. Zur Substanzfamilie der Tocomonoenole zählt das α-Tocomonoenol, das β-Tocomonoenol, das χ-Tocomonoenol und das δ-Tocomonoenol. Die Substanzfamilie der marinen Tocopherole schließt α-MDT, β- MDT, χ- MDT und δ-MDT ein und die Substanzfamilie der Tocotrienole umfasst neben α-Tocotrienol, β-Tocotrienol, χ-Tocotrienol auch δ-Tocotrienol.

Alle vorgenannten Vitamin-E-Formen verbessern die Wirkung der erfindungsgemäßen Mittel auf Zahnfleisch und Mundschleimhaut. Aufgrund ihrer Wirkung besonders bevorzugt werden jedoch die Tocopherole, insbesondere des α-Tocopherol. Bevorzugte Mund- und Zahnpflege- und - reinigungsmittel sind dadurch gekennzeichnet, dass sie, bezogen auf ihr Gesamtgewicht, 0,001 bis 1,5 Gew.%, vorzugsweise 0,01 bis 1,0 Gew.-%, und insbesondere 0,05 bis 0,5 Gew.-% gegebenenfalls acetyliertes α-Tocopherol enthalten.

Die Tocopherole weisen drei Stereozentren auf. Folglich existieren also von jedem der vier oben genannten Tocopherole (α-, β-, χ- und δ-Tocopherol) jeweils acht Stereoisomere. Als im Rahmen der vorliegenden Anmeldung besonders wirkungsvoll hat sich dass *RRR*-α-Tocopherol erwiesen. Besonders bevorzugte Mund- und Zahnpflege- und -reinigungsmittel sind daher dadurch gekennzeichnet, dass sie, bezogen auf ihr Gesamtgewicht, 0,001 bis 1,5 Gew.-%, vorzugsweise 0,01 bis 1,0 Gew.-%, und insbesondere 0,05 bis 0,5 Gew.-% gegebenenfalls acetyliertes *RRR*-α-Tocopherol enthalten.

Da Vitamin E einschließlich der Tocopherole und des α-Tocopherols eine vergleichsweise geringe Stabilität aufweisen, wird im Rahmen der vorliegenden Anmeldung der Einsatz von acetyliertem Tocopherol (Tocopherylacetat), beispielsweise acetyliertem α-Tocopherol, insbesondere acetyliertem *RRR*-α-Tocopherol bevorzugt. Bevorzugte Mund- und Zahnpflege- und -reinigungsmittel sind dadurch gekennzeichnet, dass sie, bezogen auf ihr Gesamtgewicht, 0,001 bis 1,5 Gew.-%, vorzugsweise 0,01 bis 1,0 Gew.-%, und insbesondere 0,05 bis 0,5 Gew.-% Tocopherylacetat, vorzugsweise α-Tocopherylacetat, insbesondere *RRR*-α-Tocopherylacetat enthalten.

Als wundheilende und entzündungshemmende Stoffe eignen sich beispielsweise Allantoin, Azulen, Kamillenextrakten, Tocopherol, Panthenol, Bisabolol, insbesondere jedoch Salbeiextrakte. Mund- und Zahnpflege- und -reinigungsmittel nach einem der vorherigen Ansprüche, dadurch gekennzeichnet, dass sie, bezogen auf ihr Gesamtgewicht, 0,001 bis 2,0 Gew.-%, vorzugsweise 0,01 bis 1,0 Gew.-% und insbesondere 0,05 bis 0,25 Gew.-% Salbei-Blattextrakt enthalten, werden erfindungsgemäß bevorzugt.

Ein Extrakt im Sinne der vorliegenden Anmeldung ist ein Stoff oder Stoffgemisch, welches durch Extraktion gewonnen wurde. Die Angaben zum Gewichtsanteil des Extrakts am Gesamtgewicht erfindungsgemäßer Mittel beziehen sich also auf die aus dem Extraktionsgut durch Extraktion erhaltenen Stoffe oder Stoffgemische nicht jedoch auf etwaige Hilfs- oder Begleitstoffe, wie die zur Extraktion eingesetzten Lösungsmittel.

Man unterscheidet nach der Beschaffenheit *Trockenextrakte* d.h. bis zur Trockene eingedampfte Extrakte, *Fluidextrakte* d.h. mit Lösungsmitteln so hergestellte Extrakte, daß aus einem Teil Droge höchstens 2 Teile Fluid-Extrakt gewonnen werden, *zähflüssige Extrakte bzw. Dickextrakte,* d.h. Extrakte, bei denen ein Teil des Lösungsmittels verdampft wird.

Extrakte des Salbeis werden vor allem aus den Blättern gewonnen. Erfindungsgemäß geeignet sind sämtliche Extrakte, aus Kostengründen sind Extrakte aus den Blättern.

Die erfindungsgemäß verwendeten Extrakte werden durch Extraktion vorzugsweise mit organischen Lösemitteln, Wasser oder Gemischen daraus, gewonnen. Bevorzugt geeignete organische Lösemittel sind Ketone (z.B. Aceton), Ether, Ester, Alkohole oder halogenierte Kohlenwasserstoffe. Besonders bevorzugte Extraktionsmittel sind Wasser und/oder Alkohole. Unter den Alkoholen sind dabei (C₁ bis C₆)- Alkohole, wie Ethanol und Isopropanol und zwar sowohl als alleiniges Extraktionsmittel als auch in Mischung mit Wasser, bevorzugt. Auch solventfreie Extraktionsmethoden, einschließlich der überkritischen CO₂-Extraktion sind einsetzbar.

Die erfindungsgemäßen Mund- und Zahnpflege- und -reinigungsmittel können weitere Inhaltsstoffe enthalten. Bevorzugt ist hierbei der Einsatz von sogenannten Feuchthaltemitteln, die bei Zahnpasten das Austrocknen verhindern. Bei sogenannten Flüssigzahncremes mit fließfähiger Rheologie dienen diese als Matrix und werden in höheren Mengen eingesetzt. Bei Mundwässern und Mundspülungen dienen diese Alkohole als Konsistenzregler und zusätzliche Süßungsmittel.

Hier sind erfindungsgemäße Mund- und Zahnpflege- und -reinigungsmittel bevorzugt, die - bezogen auf ihr Gewicht - 10 bis 50 Gew.-%, vorzugsweise 12,5 bis 45 Gew.%, besonders bevorzugt 15 bis 40 Gew.-%, weiter bevorzugt 17,5 bis 35 Gew.% und insbesondere 20 bis 29 Gew.% mehrwertige(n) Alkohol(e) aus der Gruppe Sorbit und/oder Glycerin und/oder 1,2-Propylenglycol oder deren Mischungen enthalten.

Für bestimmte Anwendungsbereiche kann es vorteilhaft sein, nur einen der drei oben genannten Inhaltsstoffe einzusetzen. In den meisten Fällen ist dabei Sorbit bevorzugt. Allerdings können auf anderen Anwendungsgebieten Mischungen von zwei der drei Stoffe oder aller drei Stoffe bevorzugt sein. Besonders vorteilhaft hat sich hier eine Mischung aus Glycerin, Sorbit und 1,2-Propylenglycol in einem Gewichtsverhältnis von 1 : (0,5-1): (0,1-0,5) erwiesen.

Neben Sorbit bzw. Glycerin bzw. 1,2-Propylenglycol eignen sich als weitere mehrwertige Alkohole solche mit mindestens 2 OH-Gruppen, vorzugsweise Mannit, Xylitol, Polyethylenglycol, Polypropylenglycol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt. Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden. Wichtige Vertreter von Polyhydroxyverbindungen mit 2 OH-Gruppen sind auch die Polyethylen- und Polypropylenglycole. Als bevorzugte weitere mehrwertige Alkohole können z. B. Xylit, Propylenglycole, Polyethylenglycole, insbesondere solche mit mittleren Molekulargewichten von 200-800 eingesetzt werden.

Besonders bevorzugt ist der Einsatz von Sorbit, so daß Mittel, die außer Sorbit keine anderen mehrwertigen Alkohole enthalten, besonders bevorzugt sind.

Die erfindungsgemäßen Zusammensetzungen können zusätzlich mindestens ein Poliermittel enthalten. Als Poliermittel eignen sich prinzipiell alle für Zahnpasten bekannten Reibkörper, insbesondere solche, die keine Calciumionen enthalten. Bevorzugt geeignete Poliermittel-komponenten sind daher Kieselsäuren, Aluminiumhydroxid, Aluminiumoxid, Natrium-aluminiumsilikate, organische Polymere oder Gemische solcher Reibkörper.

Calciumhaltige Polierkomponenten wie z.B. Kreide, Calciumpyrophosphat, Dicalcium-phosphatdihydrat können aber in Mengen bis zu 5 Gew.-% - bezogen auf die Gesamtzusammensetzung - enthalten sein.

Der Gesamtgehalt an Poliermitteln liegt vorzugsweise im Bereich von 5 - 50 Gew.-% des Zahnpflegemittels. Erfindungsgemäß bevorzugte Mittel enthalten Poliermittel innerhalb engerer Mengenbereiche. Hier sind erfindungsgemäß bevorzugte Mund- und Zahnpflege- und - reinigungsmittel dadurch gekennzeichnet, dass sie - bezogen auf ihr Gewicht - 1 bis 25 Gew.-%, vorzugsweise 2,5 bis 20 Gew.-%, weiter bevorzugt 5 bis 18 Gew.-% und insbesondere 7,5 bis 16 Gew.% Poliermittel enthalten.

Besonders geeignet sind Zahnpasten und flüssige Zahnreinigungsmittel, die als Poliermittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die so genannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, besonders geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Bevorzugt geeignet ist eine Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident^{®}12 DS (DEGUSSA) erhältlich. Andere Fällungskieselsäuren dieser Art sind Sident^{®} 8 (Evonik) und Sorbosil^{®} AC 39 (PQ Corporation). Diese Kieselsäuren zeichnen sich durch eine geringere Verdickungswirkung und eine etwas höhere mittlere Teilchengröße von 8 -14 µm bei einer spezifischen Oberfläche von 40 - 75 m²/g (nach BET) aus und eignen sich besonders gut für flüssige Zahncremes. Diese sollten eine Viskosität (25°C, Scherrate D = 10 s⁻¹) von 10 - 100 Pas aufweisen.

Zahnpasten, die eine deutlich höhere Viskosität von mehr als 100 Pas (25° C, D = 10 s⁻¹) aufweisen, benötigen hingegen einen genügend hohen Anteil an Kieselsäuren mit einer Teilchengröße von weniger als 5 µm, bevorzugt wenigstens 3 Gew.% einer Kieselsäure mit einer Partikelgröße von 1 - 3 µm. Solchen Zahnpasten setzt man daher bevorzugt neben den genannten Fällungskieselsäuren noch feinteiligere, so genannte Verdickungs-Kieselsäuren mit einer BET-Oberfläche von 150 - 250 m²/g zu, z.B. das Handelsprodukt Sident^{®} 22S.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend sind erfindungsgemäße Mund- und Zahnpflege- und -reinigungsmittel bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper, vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Erfindungsgemäß besonders bevorzugte Mund und Zahnpflege- und -reinigungsmittel enthalten ausschließlich Poliermittel aus der Gruppe der Kieselsäuren, Aluminiumhydroxid, Aluminiumoxid, Dicalciumphosphat-dihydrat (CaHPO₄ ·2H₂O) oder Gemische dieser Reibkörper. Diese Poliermittel haben sich als besonders effizient bei der Lösung der erfindungsgemäßen Aufgabe erwiesen.

Ganz besonders bevorzugt enthalten die erfindungsgemäßen Zusammensetzungen bezogen auf ihr Gewicht 1 bis 30 Gew.-% Fällungskieselsäure(n) mit einer spezifischen Oberfläche nach ISO 5794-1, Anhang D von ≤ 55 m²/g. Vorzugsweise werden die Fällungskieselsäuren, die entsprechende spezifische Oberflächen aufweisen, innerhalb engerer Mengenbereiche eingesetzt, und insbesondere bevorzugt werden Fällungskieselsäuren eingesetzt, die noch niedrigere spezifische Oberflächen nach ISO 5794-1, Anhang D aufweisen. Bevorzugte erfindungsgemäße Mund- und Zahnpflege- und - reinigungsmittel enthalten 2,5 bis 25 Gew.%, vorzugsweise 5 bis 20 Gew.%, besonders bevorzugt 7,5 bis 17,5 Gew.-%, weiter bevorzugt 8,0 bis 15,0 Gew.-% und insbesondere 10,0 bis 13,0 Gew.-% Fällungskieselsäure(n) mit einer spezifischen Oberfläche nach ISO 5794-1, Anhang D von ≤ 55 m²/g. Besonders bevorzugte erfindungsgemäße Mund und Zahnpflege- und -reinigungsmittel sind dadurch gekennzeichnet, dass sämtliche im Mittel enthaltenen Fällungskieselsäure(n) eine spezifische Oberfläche nach ISO 5794-1, Anhang D von ≤ 53 m²/g, vorzugsweise von ≤ 51 m²/g, weiter bevorzugt von ≤ 49 m²/g und insbesondere von ≤ 47 m²/g aufweisen.

Die erfindungsgemäße Kombination aus anionischem und amphoterem Tensid sowie CMC und Xanthan ist schonend für das Zahnfleisch und stellt gleichzeitig eine ausreichende Reinigung der Zahnoberflächen sicher.

Für die Wirkung der erfindungsgemäßen Mittel hat es sich als vorteilhaft erwiesen, die zuvor beschriebene Wirkstoffkombination durch weitere spezifische Wirkstoffe zu ergänzen, wobei sich wundheilende und entzündungshemmende Stoffe und Fluoridverbindungen als besonderes wirkungsvoll erwiesen haben.

Als wundheilende und entzündungshemmende Stoffe eignen sich beispielsweise Allantoin, Azulen, Kamillenextrakten, Tocopherol, Panthenol, Bisabolol, insbesondere jedoch Salbeiextrakte. Mund- und Zahnpflege- und -reinigungsmittel nach einem der vorherigen Ansprüche, dadurch gekennzeichnet, dass sie, bezogen auf ihr Gesamtgewicht, 0,001 bis 2,0 Gew.-%, vorzugsweise 0,01 bis 1,0 Gew.-% und insbesondere 0,05 bis 0,25 Gew.-% Salbei-Blattextrakt enthalten, werden erfindungsgemäß bevorzugt.

Ein Extrakt im Sinne der vorliegenden Anmeldung ist ein Stoff oder Stoffgemisch, welches durch Extraktion gewonnen wurde. Die Angaben zum Gewichtsanteil des Extrakts am Gesamtgewicht erfindungsgemäßer Mittel beziehen sich also auf die aus dem Extraktionsgut durch Extraktion erhaltenen Stoffe oder Stoffgemische nicht jedoch auf etwaige Hilfs- oder Begleitstoffe, wie die zur Extraktion eingesetzten Lösungsmittel.

Man unterscheidet nach der Beschaffenheit *Trockenextrakte* d.h. bis zur Trockene eingedampfte Extrakte, *Fluidextrakte* d.h. mit Lösungsmitteln so hergestellte Extrakte, daß aus einem Teil Droge höchstens 2 Teile Fluid-Extrakt gewonnen werden, *zähflüssige Extrakte bzw. Dickextrakte,* d.h. Extrakte, bei denen ein Teil des Lösungsmittels verdampft wird.

Extrakte des Salbeis werden vor allem aus den Blättern gewonnen. Erfindungsgemäß geeignet sind sämtliche Extrakte, aus Kostengründen sind Extrakte aus den Blättern.

Die erfindungsgemäß verwendeten Extrakte werden durch Extraktion vorzugsweise mit organischen Lösemitteln, Wasser oder Gemischen daraus, gewonnen. Bevorzugt geeignete organische Lösemittel sind Ketone (z.B. Aceton), Ether, Ester, Alkohole oder halogenierte Kohlenwasserstoffe. Besonders bevorzugte Extraktionsmittel sind Wasser und/oder Alkohole. Unter den Alkoholen sind dabei (C₁ bis C₆)- Alkohole, wie Ethanol und Isopropanol und zwar sowohl als alleiniges Extraktionsmittel als auch in Mischung mit Wasser, bevorzugt. Auch solventfreie Extraktionsmethoden, einschließlich der überkritischen CO₂-Extraktion sind einsetzbar.

Erfindungsgemäße Mittel können als Zahnpasten oder Zahncremes formuliert werden. Ein weiterer Gegenstand der vorliegenden Erfindung ist die Verwendung von erfindungsgemäßen Mitteln zur Reinigung von Zähnen mittels manuell betätigter oder elektrischer Zahnbürsten. Im Falle elektrischer Zahnbürsten besitzen die erfindungsgemäßen Mittel den weiteren Vorteil, daß sie bereits in geringen Mengen wirksam sind und darüber hinaus die Mechanik des elektrischen Bürstenkopfes nicht beeinträchtigen.

Ein weiterer Gegenstand der vorliegenden Erfindung ist ein Verfahren zum Reinigen von Zähnen, dadurch gekennzeichnet, daß ein erfindungsgemäßes Mittel auf den Bürstenkopf einer elektrischen Zahnbürste aufgetragen und mit der elektrischen Zahnbürste die Zähne geputzt werden.

Ein weiterer Gegenstand der vorliegenden Erfindung ist ein Verfahren zur Zahnreinigung, gekennzeichnet durch die Schritte
a) Bereitstellung einer Zahnbürste, deren Bürstenkopf elektrisch in Bewegung versetzt werden kann;
b) Applikation von 0,5 bis 5 g eines Mittels nach einem der Ansprüche 1 bis 8 auf den Bürstenkopf,
c) 30- bis 300-sekündiges Zähneputzen mit dem Mittel nach einem der Ansprüche 1 bis 8 unter Einsatz des elektrisch in Bewegung versetzten Bürstenkopfes.

Bezüglich bevorzugter Ausführungsformen des erfindungsgemäßen Verfahrens gilt mutatis mutandis das zu den erfindungsgemäßen Mitteln Gesagte.

Durch die erfindungsgemäße Viererkombination können in Mund- und Zahnpflege- und -reinigungsmitteln enthaltene Aktivsubstanzen wie z.V. Vitamin E oder antibakterielle Verbindungen effektiv auf die Zahnoberfläche sowie das Zahnfleisch und die Mundschleimhaut aufgebracht und dort deponiert werden.

Erfindungsgemäße Mittel können als Zahnpasten oder Zahncremes formuliert werden. Ein weiterer Gegenstand der vorliegenden Erfindung ist die Verwendung von erfindungsgemäßen Mitteln zur Reinigung von Zähnen mittels manuell betätigter oder elektrischer Zahnbürsten. Im Falle elektrischer Zahnbürsten besitzen die erfindungsgemäßen Mittel den weiteren Vorteil, daß sie bereits in geringen Mengen wirksam sind und darüber hinaus die Mechanik des elektrischen Bürstenkopfes nicht beeinträchtigen.

Ein weiterer Gegenstand der vorliegenden Erfindung ist ein Verfahren zum Reinigen von Zähnen, dadurch gekennzeichnet, daß ein erfindungsgemäßes Mittel auf den Bürstenkopf einer elektrischen Zahnbürste aufgetragen und mit der elektrischen Zahnbürste die Zähne geputzt werden.

Ein weiterer Gegenstand der vorliegenden Erfindung ist ein Verfahren zur Zahnreinigung, gekennzeichnet durch die Schritte
a) Bereitstellung einer Zahnbürste, deren Bürstenkopf elektrisch in Bewegung versetzt werden kann;
b) Applikation von 0,5 bis 5 g eines Mittels nach einem der Ansprüche 1 bis 8 auf den Bürstenkopf,
c) 30- bis 300-sekündiges Zähneputzen mit dem Mittel nach einem der Ansprüche 1 bis 8 unter Einsatz des elektrisch in Bewegung versetzten Bürstenkopfes.

Bezüglich bevorzugter Ausführungsformen des erfindungsgemäßen Verfahrens gilt mutatis mutandis das zu den erfindungsgemäßen Mitteln Gesagte.

Durch die erfindungsgemäße Viererkombination können in Mund- und Zahnpflege- und -reinigungsmitteln enthaltene Aktivsubstanzen wie z.V. Vitamin E oder antibakterielle Verbindungen effektiv auf die Zahnoberfläche sowie das Zahnfleisch und die Mundschleimhaut aufgebracht und dort deponiert werden.

Ein weiterer Gegenstand der vorliegenden Erfindung ist daher die nicht-therapeutische, kosmetische Verwendung von Mischungen aus
a) 0,05 bis 5,0 Gew.-%, anionische(m/n) Tensid(en), vorzugsweise Natriumlaurylsulfat;
b) 0,05 bis 5,0 Gew.-%, amphotere(m/n) Tensid(en), vorzugsweise Cocamidopropylbetain;
c) 0,1 bis 10 Gew.-% Carboxymethylcellulose;
d) 0,1 bis 10 Gew.-% Xanthan
zur Steigerung der Verfügbarkeit von Aktivsubstanzen in Mund- und Zahnpflege- und - reinigungsmitteln.

Auch bezüglich bevorzugter Ausführungsformen dieser erfindungsgemäßen Verwendung gilt ebenfalls mutatis mutandis das zu den erfindungsgemäßen Mitteln Gesagte.

## Patentansprüche

1. Mund- und Zahnpflege- und -reinigungsmittel, enthaltend - bezogen auf sein Gewicht -
a) 0,05 bis 5,0 Gew.-% Natriumlaurylsulfat;
b) 0,05 bis 5,0 Gew.-% Cocamidopropylbetain;
c) 0,1 bis 10 Gew.-% Carboxymethylcellulose;
d) 0,1 bis 10 Gew.-% Xanthan,
**dadurch gekennzeichnet, daß** das Gewichtsverhältnis von anionischen Tensiden zu amphoteren Tensiden bei ≥ 4:1 liegt.

2. Mund- und Zahnpflege- und -reinigungsmittel nach Anspruch 1, **dadurch gekennzeichnet, daß** es 0,1 bis 4,0 Gew.-%, vorzugsweise 0,75 bis 3,0 Gew.-%, besonders bevorzugt 1,0 bis 2,5 Gew.-%, weiter bevorzugt 1,25 bis 2,0 Gew.-% und insbesondere 1,5 bis 1,8 Gew.-% Natriumlaurylsulfat enthält.

3. Mund und Zahnpflege- und -reinigungsmittel nach einem der Ansprüche 1 oder 2, **dadurch gekennzeichnet, daß** es 0,1 bis 4,0 Gew.-%, vorzugsweise 0,2 bis 3,0 Gew.-%, besonders bevorzugt 0,3 bis 2,5 Gew.-%, weiter bevorzugt 0,4 bis 2,0 Gew.-% und insbesondere 0,5 bis 1,0 Gew.-% Cocamidopropylbetain enthält.

4. Mund und Zahnpflege- und -reinigungsmittel nach einem der Ansprüche 1 bis 3 **dadurch gekennzeichnet, daß** das Gewichtsverhältnis von anionischen Tensiden zu amphoteren Tensiden bei ≥ 5:1 , und insbesondere bei ≥ 7:1 liegt.

5. Mund- und Zahnpflege- und -reinigungsmittel nach einem der Ansprüche 1 bis 4, **dadurch gekennzeichnet, daß** es - bezogen auf sein Gewicht - 0,2 bis 7,5 Gew.-%, vorzugsweise 0,25 bis 5 Gew.-%, besonders bevorzugt 0,3 bis 4 Gew.-%, weiter bevorzugt 0,4 bis 3 Gew.-%, noch weiter bevorzugt 0,45 bis 2 Gew.-% und insbesondere 0,5 bis 0,8 Gew.-% Carboxymethylcellulose enthält.

6. Mund- und Zahnpflege- und -reinigungsmittel nach einem der Ansprüche 1 bis 5, **dadurch gekennzeichnet, daß** es Carboxymethylcellulose(n) enthält, welche in einer 2 Gew.-%igen wäßrigen Lösung bei 20°C eine Viskosität von 1.000 bis 100.000 mPas, vorzugsweise 2.000 bis 50.000 mPas und insbesondere 5.000 bis 25.000 mPa·s mit einem Rotationsviskosimeter bei einer Scherrate von 2,55 1/s aufweist/aufweisen.

7. Mund- und Zahnpflege- und -reinigungsmittel nach einem der Ansprüche 1 bis 6, **dadurch gekennzeichnet, daß** es Carboxymethylcellulose(n) enthält, welche in einer 2 Gew.-%igen wäßrigen Lösung bei 20°C eine Viskosität von 5.000 bis 100.000 mPas, vorzugsweise 10.000 bis 70.000 mPas und insbesondere 35.000 bis 50.000 mPa·s mit einem Brookfield LVT Viskosimeter aufweist/aufweisen.

8. Mund- und Zahnpflege- und -reinigungsmittel nach einem der Ansprüche 1 bis 7, **dadurch gekennzeichnet, daß** es Carboxymethylcellulose(n) enthält, welche in einer 2 Gew.-%igen wäßrigen Lösung bei 20°C eine Viskosität von 50.000 bis 250.000 mPas, vorzugsweise 75.000 bis 200.000 mPas und insbesondere 120.000 bis 140.000 mPas mit einem Höppler Viskosimeter aufweist/aufweisen.

9. Mund- und Zahnpflege- und -reinigungsmittel nach einem der Ansprüche 1 bis 8, **dadurch gekennzeichnet, daß** es bei 20°C eine Viskosität (gemessen mit Brookfield Synchro-Lectric Viskosimeter, Typ RVT mit Helipath-Stativ, Spindel 3 und 20 U/min) von 100 bis 1000 Pas (100.000 bis 1.000.000 mPas), vorzugsweise von 200 bis 750 Pas (200.000 bis 750.000 mPas), weiter bevorzugt von 350 bis 650 Pas (350.000 bis 650.000 mPas) und insbesondere von 450 bis 550 Pas (450.000 bis 550.000 mPas) aufweist.

10. Mund- und Zahnpflege- und -reinigungsmittel nach einem der Ansprüche 1 bis 9, **dadurch gekennzeichnet, daß** es - bezogen auf sein Gewicht - 0,15 bis 5 Gew.-%, vorzugsweise 0,2 bis 2,5 Gew.-%, besonders bevorzugt 0,25 bis 1 Gew.-%, weiter bevorzugt 0,3 bis 0,75 Gew.-%, noch weiter bevorzugt 0,35 bis 0,6 Gew.-% und insbesondere 0,4 bis 0,5 Gew.-% Xanthan enthält.

11. Mund- und Zahnpflege- und -reinigungsmittel nach einem der Ansprüche 1 bis 10, **dadurch gekennzeichnet, daß** das Gewichtsverhältnis von Carboxymethylcellulose zu Xanthan zwischen 2:1 und 1:2, vorzugsweise zwischen 1,75:1 und 1:1,5, besonders bevorzugt zwischen 1,5:1 und 1:1,25 und insbesondere zwischen 1,2:1 und 1:1 liegt.

12. Mund- und Zahnpflege- und -reinigungsmittel nach einem der Ansprüche 1 bis 11, **dadurch gekennzeichnet, daß** es - bezogen auf sein Gewicht -10 bis 50 Gew.-%, vorzugsweise 12,5 bis 45 Gew.-%, besonders bevorzugt 15 bis 40 Gew.-%, weiter bevorzugt 17,5 bis 35 Gew.-% und insbesondere 20 bis 29 Gew.-% mehrwertige(n) Alkohol(e) aus der Gruppe Sorbit und/oder Glycerin und/oder 1,2-Propylenglycol.-%, jeweils bezogen auf das Gewichts des gesamten Mittels, enthält.

13. Verfahren zur Zahnreinigung, **gekennzeichnet durch** die Schritte
a) Bereitstellung einer Zahnbürste, deren Bürstenkopf elektrisch in Bewegung versetzt werden kann;
b) Applikation von 0,5 bis 5 g eines Mittels nach einem der Ansprüche 1 bis 12 auf den Bürstenkopf,
c) 30- bis 300-sekündiges Zähneputzen mit dem Mittel nach einem der Ansprüche 1 bis 8 unter Einsatz des elektrisch in Bewegung versetzten Bürstenkopfes.

## Claims

1. An oral and dental care and cleaning agent, containing, based on its weight,
a) 0.05 to 5.0 wt.% sodium lauryl sulfate;
b) 0.05 to 5.0 wt.% cocamidopropyl betaine;
c) 0.1 to 10 wt.% carboxymethyl cellulose;
d) 0.1 to 10 wt.% xanthan gum,
**characterized in that** the weight ratio of anionic surfactants to amphoteric surfactants is ≥ 4:1.

2. The oral and dental care and cleaning agent according to claim 1, **characterized in that** it contains 0.1 to 4.0 wt.%, preferably 0.75 to 3.0 wt.%, particularly preferably 1.0 to 2.5 wt.%, more preferably 1.25 to 2.0 wt.%, and in particular 1.5 to 1.8 wt.%, sodium lauryl sulfate.

3. The oral and dental care and cleaning agent according to claim 1 or 2, **characterized in that** it contains 0.1 to 4.0 wt.%, preferably 0.2 to 3.0 wt.%, particularly preferably 0.3 to 2.5 wt.%, more preferably 0.4 to 2.0 wt.%, and in particular 0.5 to 1.0 wt.%, cocamidopropyl betaine.

4. The oral and dental care and cleaning agent according to one of claims 1 to 3, **characterized in that** the weight ratio of anionic surfactants to amphoteric surfactants is ≥ 5:1, and in particular ≥ 7:1.

5. The oral and dental care and cleaning agent according to one of claims 1 to 4, **characterized in that** it contains, based on its weight, 0.2 to 7.5 wt.%, preferably 0.25 to 5 wt.%, particularly preferably 0.3 to 4 wt.%, more preferably 0.4 to 3 wt.%, even more preferably 0.45 to 2 wt.%, and in particular 0.5 to 0.8 wt.%, carboxymethyl cellulose.

6. The oral and dental care and cleaning agent according to one of claims 1 to 5, **characterized in that** it contains carboxymethyl cellulose(s), which, in a 2 wt.% aqueous solution at 20°C, has/have a viscosity of from 1,000 to 100,000 mPas, preferably 2,000 to 50,000 mPas, and in particular 5,000 to 25,000 mPa·s, using a rotation viscometer at a shear rate of 2.55 1/s.

7. The oral and dental care and cleaning agent according to one of claims 1 to 6, **characterized in that** it contains carboxymethyl cellulose(s), which, in a 2 wt.% aqueous solution at 20°C, has/have a viscosity of from 5,000 to 100,000 mPas, preferably 10,000 to 70,000 mPas, and in particular 35,000 to 50,000 mPa·s, using a Brookfield LVT viscometer.

8. The oral and dental care and cleaning agent according to one of claims 1 to 7, **characterized in that** it contains carboxymethyl cellulose(s), which, in a 2 wt.% aqueous solution at 20°C, has/have a viscosity of from 50,000 to 250,000 mPas, preferably 75,000 to 200,000 mPas, and in particular 120,000 to 140,000 mPas, using a Höppler viscometer.

9. The oral and dental care and cleaning agent according to one of claims 1 to 8, **characterized in that** it has, at 20°C, a viscosity (as measured using a Brookfield Synchro-Lectric viscometer, RVT type having the Helipath Stand, spindle 3 and 20 rpm) of from 100 to 1,000 Pas (100,000 to 1,000,000 mPas), preferably 200 to 750 Pas (200,000 to 750,000 mPas), more preferably 350 to 650 Pas (350,000 to 650,000 mPas), and in particular 450 to 550 Pas (450,000 to 550,000 mPas).

10. The oral and dental care and cleaning agent according to one of claims 1 to 9, **characterized in that** it contains, based on its weight, 0.15 to 5 wt.%, preferably 0.2 to 2.5 wt.%, particularly preferably 0.25 to 1 wt.%, more preferably 0.3 to 0.75 wt.%, even more preferably 0.35 to 0.6 wt.%, and in particular 0.4 to 0.5 wt.%, xanthan gum.

11. The oral and dental care and cleaning agent according to one of claims 1 to 10, **characterized in that** the weight ratio of carboxymethyl cellulose to xanthan gum is between 2:1 and 1:2, preferably between 1.75:1 and 1:1.5, particularly preferably between 1.5:1 and 1:1.25, and in particular between 1.2:1 and 1:1.

12. The oral and dental care and cleaning agent according to one of claims 1 to 11, **characterized in that** it contains, based on its weight, 10 to 50 wt.%, preferably 12.5 to 45 wt.%, particularly preferably 15 to 40 wt.%, more preferably 17.5 to 35 wt.%, and in particular 20 to 29 wt.%, polyvalent alcohol(s) from the group consisting of sorbitol and/or glycerol and/or 1,2-propylene glycol, in each case based on the weight of the total agent.

13. A method for cleaning teeth, **characterized by** the steps of:
a) providing a toothbrush, the brush head of which can be set in motion electrically;
b) applying 0.5 to 5 g of an agent according to one of claims 1 to 12 to the brush head;
c) brushing the teeth with the agent according to one of claims 1 to 8 for 30 to 300 seconds using the brush head that is set in motion electrically.

## Revendications

1. Produit de lavage et de nettoyage de la bouche et des dents contenant, rapporté à son poids :
a) 0,05 à 5,0 % en poids de laurylsulfate de sodium,
b) 0,05 à 5,0 % en poids de cocamidopropyl bétaïne,
c) 0,1 à 10 % en poids de carboxyméthylcellulose,
d) 0,1 à 10 % en poids de xanthane,
**caractérisé en ce que** le rapport pondéral des tensioactifs anioniques aux tensioactifs amphotères est ≥ 4:1.

2. Produit de lavage et de nettoyage de la bouche et des dents selon la revendication 1, **caractérisé en ce qu'**il contient 0,1 à 4,0 % en poids, de préférence 0,75 à 3,0 % en poids, particulièrement préférentiellement 1,0 à 2,5 % en poids, plus préférentiellement 1,25 à 2,0 % en poids et en particulier 1,5 à 1,8 % en poids de laurylsulfate de sodium.

3. Produit de lavage et de nettoyage de la bouche et des dents selon une des revendications 1 ou 2, **caractérisé en ce qu'**il contient 0,1 à 4,0 % en poids, de préférence 0,2 à 3,0 % en poids, particulièrement préférentiellement 0,3 à 2,5 % en poids, plus préférentiellement 0,4 à 2,0 % en poids et en particulier 0,5 à 1,0 % en poids de cocamidopropyl bétaïne.

4. Produit de lavage et de nettoyage de la bouche et des dents selon une des revendications 1 à 3, **caractérisé en ce que** le rapport pondéral des tensioactifs anioniques aux tensioactifs amphotères est ≥ 5:1, et en particulier ≥ 7:1.

5. Produit de lavage et de nettoyage de la bouche et des dents selon une des revendications 1 à 4, **caractérisé en ce qu'**il contient, rapporté à son poids, 0,2 à 7,5 % en poids, de préférence 0,25 à 5 % en poids, particulièrement préférentiellement 0,3 à 4 % en poids, plus préférentiellement 0,4 à 3 % en poids, encore plus préférentiellement 0,45 à 2 % en poids et en particulier 0,5 à 0,8 % en poids de carboxyméthylcellulose.

6. Produit de lavage et de nettoyage de la bouche et des dents selon une des revendications 1 à 5, **caractérisé en ce qu'**il contient une ou plusieurs carboxyméthylcelluloses présentant, dans une solution aqueuse à 2 % en poids à 20°C, une viscosité de 1 000 à 100 000 mPa.s, de préférence de 2 000 à 50 000 mPa.s et en particulier de 5 000 à 25 000 mPa.s mesurée avec un viscosimètre à rotation à un taux de cisaillement de 2,55 s⁻¹.

7. Produit de lavage et de nettoyage de la bouche et des dents selon une des revendications 1 à 6, **caractérisé en ce qu'**il contient une ou plusieurs carboxyméthylcelluloses présentant, dans une solution aqueuse à 2 % en poids à 20°C, une viscosité de 5 000 à 100 000 mPa.s, de préférence de 10 000 à 70 000 mPa.s et en particulier de 35 000 à 50 000 mPa.s mesurée avec un viscosimètre Brookfield LVT.

8. Produit de lavage et de nettoyage de la bouche et des dents selon une des revendications 1 à 7, **caractérisé en ce qu'**il contient une ou plusieurs carboxyméthylcelluloses présentant, dans une solution aqueuse à 2 % en poids à 20°C, une viscosité de 50 000 à 250 000 mPa.s, de préférence de 75 000 à 200 000 mPa.s et en particulier de 120 000 à 140 000 mPa.s mesurée avec un viscosimètre Höppler.

9. Produit de lavage et de nettoyage de la bouche et des dents selon une des revendications 1 à 8, **caractérisé en ce qu'**il prés000 ente à 20°C une viscosité (mesurée par un viscosimètre Brookfield Synchro-Lectric type RVT avec Helipath-Stativ, broche 3 à 20 tr/min) de 100 à 1 000 Pa.s (100 000 à 1 000 000 mPa.s), de préférence de 200 à 750 Pa.s (200 000 à 750 000 mPa.s), plus préférentiellement de 350 à 650 Pa.s (350 000 à 650 000 mPa.s) et en particulier de 450 à 550 Pa.s (450 000 à 550 000 mPa.s).

10. Produit de lavage et de nettoyage de la bouche et des dents selon une des revendications 1 à 9, **caractérisé en ce qu'**il contient, rapporté à son poids, 0,15 à 5 % en poids, de préférence 0,2 à 2,5 % en poids, particulièrement préférentiellement 0,25 à 1 % en poids, plus préférentiellement 0,3 à 0,75 % en poids, encore plus préférentiellement 0,35 à 0,6 % en poids et en particulier 0,4 à 0,5 % en poids de xanthane.

11. Produit de lavage et de nettoyage de la bouche et des dents selon une des revendications 1 à 10, **caractérisé en ce que** le rapport pondéral carboxyméthylcellulose/xanthane est entre 2:1 et 1:2, de préférence entre 1,75:1 et 1:1,5, particulièrement préférentiellement entre 1,5:1 et 1:1,25 et en particulier entre 1,2:1 et 1:1.

12. Produit de lavage et de nettoyage de la bouche et des dents selon une des revendications 1 à 11, **caractérisé en ce qu'**il contient, rapporté à son poids, 10 à 50 % en poids, de préférence 12,5 à 45 % en poids, particulièrement préférentiellement 15 à 40 % en poids, plus préférentiellement 17,5 à 35 % en poids, et en particulier 20 à 29 % en poids d'un ou plusieurs polyols issus du groupe constitué du sorbitol et/ou de la glycérine et/ou du 1,2-propylène glycol, chacun rapporté au poids du produit total.

13. Procédé de nettoyage des dents, **caractérisé par** les étapes suivantes :
a) préparation de la brosse à dent, dont la tête peut être mise en mouvement électriquement,
b) application de 0,5 à 5 g d'un produit selon une des revendications 1 à 12 sur la tête de la brosse,
c) brossage des dents de 30 à 300 s avec le produit selon une des revendications 1 à 8 en utilisant la tête de la brosse mise en mouvement électriquement.
